# EUROPEAN PATENT SPECIFICATION

(11) **EP 1 247 526 B1**
(45) Date of publication and mention of the grant of the patent: **14.12.2005**
(21) Application number: 00900174.4
(22) Date of filing: 11.01.2000
(51) Int. Cl.: A61K 33/10, A61K 9/08, A61P 27/02

(54) **PERFUSION LIQUID PREPARATIONS FOR OPHTHALMIC OPERATIONS**
PERFUSIONSLÖSUNG ZUSAMMENSETZUNGEN FÜR OPHTHALMISCHE VERFAHREN
PREPARATIONS LIQUIDES PERFUSABLES POUR INTERVENTIONS OPHTALMIQUES

(43) Date of publication of application: 09.10.2002
(73) Proprietor: Ophtecs Corporation, Osaka-shi, Osaka 550-0002 (JP)
(72) Inventor: YONEDA, Toyoaki, Osaka-shi, Osaka 550-0002 (JP); NAKAYAMA, Yoshio, Toyooka-shi, Hyogo 668-0831 (JP); MIICHI, Hiroaki, Toyooka-shi, Hyogo 668-0831 (JP); SAITO, Fumio, Toyooka-shi, Hyogo 668-0831 (JP); NAKAMURA, Shigeru, Toyooka-shi, Hyogo 668-0831 (JP)
(74) Representative: Hallybone, Huw George
(86) International application number: PCT/JP2000/000081
(87) International publication number: WO 2001/051065

(56) References cited:
- EP-A- 0 517 970
- JP-A- 11 005 737
- US-A- 4 238 482
- US-A- 5 104 663
- US-A- 5 380 537

## Description

### Technical Field

The present invention relates to a one solution-pack preparation of irrigating solution for ocular surgery which is excellent in a living body safety and preparation stability. More specifically, it relates to a one solution-pack preparation of irrigating solution for ocular surgery which is suitable for protecting intraocular tissues, removing the substances left in the eye after surgery by suction and preventing the corneal epithelium and the conjunctiva from drying, and it is in order to perform the surgery of cataract, intraocular lens implantation, glaucoma, vitrectomy or penetrating keratoplasty and the like safely and effectively.

### Background Art

Recently, development of ocular surgery methods for surgery of cataract, intraocular lens implantation, glaucoma and the like has remarkably progressed. An irrigating solution which is used as a surgical adjuvant plays an important role in order to perform the above surgery safely and effectively. For example, when the periphery of the cornea is incised by a scalpel at the start of the surgery of cataract, the aqueous humor flows out from the anterior chamber immediately. Therefore, injection of an irrigating solution and a viscoelastic substance is indispensable for protecting intraocular tissues and cells and for maintaining the space of the anterior chamber. Further, when the irrigating solution is present in the eye at the time of breaking the opaque crystalline lens into pieces and extracting them, the pieces can be removed smoothly by suction. The irrigating solution is also used for preventing the surface of the cornea from drying during surgery.

Some important points in the preparation for achieving such application purposes of the irrigating solution are as follows: 1) the osmotic pressure and pH of the preparation must be physiologically harmonized with the intraocular tissues and the corneal endothelial cell; 2) aqueous humor components such as inorganic salts and energy sources must be added; 3) the preparation must be biologically safe; and 4) the preparation must be able to be preserved at room temperature for the long term.

Preparations of irrigating solution for ocular surgery which are currently available in the market include a commercial product containing oxyglutathione as an active ingredient and another commercial product having a bicarbonate ion-based buffer system. However, since these are a two-pack preparation which requires two types of solutions to be mixed prior to use and contain no ingredients effective for protecting the corneal endothelial cells, they have not a few problems to be solved with regard to stability, simplicity at the time of use and efficacy as a preparation.

A preparation of irrigating solution for ocular surgery which is composed essentially of 3-hydroxybutyric acid as an energy source is disclosed in the specifications of U.S. Patent Nos. 5,116,868 and 5,298,487. However, the preparations described in the specifications do not contain bicarbonate ions necessary to sustain the function of the corneal endothelial cell. The reasons disclosed in the specifications why the preparation does not have to contain the bicarbonate ions in advance are that 3-hydroxybutyric acid generates CO₂ through metabolism and the CO₂ changes into the bicarbonate ions which are therefore supplied automatically, and that when the bicarbonate ions exist in the preparation, the pH of the preparation fluctuates due to a CO₂ partial pressure, whereby the preparation becomes unstable. That is, it is disclosed that the bicarbonate ions do not have to be contained in the preparation in advance.

D-3-hydroxybutyric acid or its salts used as active ingredients in the present invention are known to be biological substances existing in humans and most of other mammals, biosynthesized through an oxidation process of fatty acids in the liver, and carried into the cornea as well as peripheral tissues out of the liver by blood to be utilized as an efficient energy source (refer to Lehninger, NEW BIOCHEM., 2nd Ed., p625, 1993 and NATURE, No. 4,841, p597, 1962). It is also known that the substances are much more useful for corneal tissues as an energy source than glucose (refer to TRANSPLANTATION, 57, p1778-1785, 1994). As for the application of D-3-hydroxybutyric acid and its salts to drugs, it has been reported that they are mixed into an infusion solution for supplying nutrients to patients in the accelerated state as to biological protein catabolism or those having an invaded body (refer to Japanese Patent Laid-Open Publication No. 2-191212).

Japanese Patent Laid-Open Publication No. 11-5737 discloses a preparation of irrigating solution for ocular surgery which comprises at least one compound selected from the group consisting of D-3-hydroxybutyric acid and its salts and bicarbonates.

### Disclosure of The Invention

It is an object of the present invention to provide a one solution-pack preparation of irrigating solution for ocular surgery of cataract, intraocular lens implantation, glaucoma, vitrectomy or penetrating keratoplasty, which is excellent in the protection of ocular tissues and endothelial cells during and after the surgery and has excellent in a living body safety.

It is another object of the present invention is to provide a one solution-pack preparation of irrigating solution for ocular surgery which not only has the above excellent properties but also is stable under severe temperature conditions.

Other objects and advantages of the present invention will become apparent from the following description.

According to the present invention, the above objects and advantages can be attained by a one solution-pack preparation of irrigating solution for ocular surgery, which comprises
(i) at least one compound selected from the group consisting of D-3-hydroxybutyric acid and its salts,
(ii) bicarbonates, and
(iii) calcium glycerophosphate.

### Brief Description of the Drawings

Fig. 1 shows the protection effects (in vitro experiments) of the optical isomers of 3-hydroxybutyric acid (to be sometimes referred to "3-HBA" hereinafter) on the corneas.
Fig. 2 shows the protection effects (in vitro experiments) of glycerophosphate ions and phosphate ions on the corneas.
Fig. 3 shows the protection effect (in vitro experiments) of calcium glycerophosphate on the cornea.
Fig. 4 shows the protection effect (in vitro experiments) of bicarbonate ions on the cornea.
Fig. 5 shows the protection effects (in vitro experiments) of D-3-HBA·Na and glucose on the corneas.
Fig. 6 shows the amounts of changes in the corneal thickness with time by the solution of the present invention and a comparative solution.
Fig. 7 shows an electroretinogram (to be referred to as "ERG" hereinafter) showing the a-wave implicit time with time of the solution of the present invention and a comparative solution.
Fig. 8 shows an ERG showing the b-wave implicit time with time of the solution of the present invention and a comparative solution.

### Detailed Description of the Invention

The preparation of the present invention will be described in detail hereinafter.

As for the absolute configuration at the C₃ position in the chemical structural formula of 3-hydroxybutyric acid, there is a group of a D-form, a DL-form and an L-form. Of these, in the present invention, the D-form is used to maximize the effectiveness of a preparation of irrigating solution for ocular surgery. This is because when the difference in activity based on the optical isomers with regard to an effect of activating the corneal endothelial cell, which is one of methods for evaluating the effectiveness of the preparation, is studied, activity is obtained with the D-form while it is hardly obtained with the L-form, and because less activity is obtained with the DL-form than with the D-form since activity is not obtained with the L-form.

Since D-3-hydroxybutyric acid and its salts can be synthesized with ease and at high asymmetric yields by asymmetrically hydrogenating the ketone groups of an acetoacetic ester in the presence of a ruthenium-optically active phosphine complex as a catalyst and subjecting the ester to alkaline hydrolysis, the compounds can be obtained at relatively low cost (refer to Japanese Patent Publication No. 6-99367.

The salts of D-3-hydroxybutyric acid in the present invention are preferably water-soluble salts, as exemplified by sodium salts, potassium salts, barium salts, magnesium salts, lithium salts, L-lysine salts, L-histidine salts and L-arginine salts.

D-3-hydroxybutyric acid and its water-soluble salts can be used solely or in combination of two or more.

In the present invention, the concentration of D-3-hydroxybutyric acid and/or its salts in the aqueous solution preparation is preferably at least 0.01 mM but less than 200 mM, more preferably at least 1 mM but less than 100 mM, and much more preferably at least 5 mM but less than 40 mM.

In the present invention, the addition of bicarbonates exerts a remarkable influence on a cornea-swelling-inhibiting effect, which is one of important indexes for sustaining corneal functions. Actually, when the corneoscleral pieces extracted from rabbits were incubated by using a preparation containing bicarbonates or no bicarbonates respectively, and changes in the corneal thicknesses were measured, the preparation containing bicarbonates restored the cornea to a nearly normal condition, whereas the preparation containing no bicarbonates accelerated the corneal swelling, proving that it was lacking in the effect of protecting the cornea. Further, the above was also true in an in vivo experiment using the eyes of rabbits; that is, the preparation containing bicarbonates restored the cornea to a normal condition, whereas the preparation containing no bicarbonates accelerated the corneal swelling.

In consideration of the above facts, bicarbonates (bicarbonate ions) must be contained in the D-3-hydroxybutiryic acid-containing preparation of the present invention.

The bicarbonates are preferably sodium bicarbonate and potassium bicarbonate. The concentration of the bicarbonates is preferably at least 0.1 mM but less than 100 mM, more preferably at least 1 mM but less than 60 mM, and much more preferably at least 10 mM but less than 60 mM, as bicarbonate ions (HCO₃⁻).

In the present invention, in place of phosphate ions and calcium ions which are aqueous humor components necessary as the components of the irrigating solution, calcium glycerophosphate is used for the purpose of improving the preparation stability under severe temperature conditions. Although glycerophosphate ions are hardly dissociated into phosphate ions in an aqueous solution under normal conditions, it has been confirmed that the glycerophosphate ions can sufficiently exhibit the same effect as the phosphate ions do. Further, in an aqueous solution under severe temperature conditions, while sometimes the phosphate ions are reacted with the calcium ions to cause precipitation, the glycerophosphate ions never cause such precipitation. In addition, it has also been found that the coexistence of D-3-hydroxybutyric acid ions and the glycerophosphate ions can further improve the retention of the function of the cornea. The concentration of calcium glycerophosphate is preferably at least 0.1 mM but less than 50 mM, and more preferably at least 1 mM but less than 10 mM. Inevitably, in the present invention, the calcium ions are contained in the same concentration in which the glycerophosphate ions are contained.

The preparation of the present invention preferably contains glucose as an additional energy source. In the present invention, D-3-hydroxybutyric acid and/or its salts are used as energy sources, and it has been confirmed that when they are used in the co-presence of glucose, a cornea-activating effect is enhanced more than when they are used alone. The concentration of glucose is preferably at least 0.01 mM but less than 50 mM, and more preferably at least 0.1 mM but less than 20 mM.

It is preferable that the preparation of present invention further contain citric acid and its salts (citrate ions). The citrate ions are added as a pH adjuster and a stabilizer. Although the citrate ions are preferably supplied as citric acid, they may also be supplied in the form of sodium citrate or potassium citrate. In the present invention, the citrate ions, in combination with bicarbonate salts, also serve as a buffer. In the present invention, when citric acid is used, there is an advantage that the final fine adjustment of pH is not needed in preparing the preparation. However, even if the citrate ions are used in the form of citrate, there is no problem in making the final fine adjustment of pH. The concentration of the citrate ions is at least 0.01 mM but less than 50 mM, and more preferably at least 0.1 mM but less than 10 mM. When the concentration of the citrate ions is set to be within the above range, the pH does not seem to be changed much during observed at 40° C and a relative humidity of 75% for 6 months.

As for the compounds used in the irrigating solution preparation in the present invention, inorganic salts present in human aqueous humor, isotonic agents and buffers for harmonizing the osmotic pressure and the pH with the intraocular tissues and the corneal endothelial cells, stabilizers and the like are preferably used as appropriate.

As the inorganic salts and isotonic agents used in the present invention, inorganic salts such as the above calcium salts, magnesium salts and alkaline metal salts, e.g., sodium chloride and potassium chloride, and isotonic agents such as carbohydrates, e.g., mannitol, sorbitol, xylitol and dextran, are preferably used. The magnesium salts are added as salts comprising the D-3-hydroxybutyric acid ions and bicarbonate ions as described above and magnesium, or as other water-soluble salts such as magnesium chloride and magnesium sulfate. The concentration of the magnesium salts is preferably at least 0.01 mM but less than 50 mM, more preferably at least 0.1 mM but less than 20 mM, much more preferably at least 0.5 mM but less than 10 mM. The concentration of the above inorganic salts and that of the above isotonic agents are preferably at least 0.01 mM but less than 1,000 mM. In addition, the osmotic pressure of the preparation is sustained preferably within the range of 270 to 350 mOsm. These can be used solely or in combination of two or more.

The pH of the aqueous solution preparation of the present invention is preferably in the range of 6.8 to 8.2, within which no damages are caused on intraocular tissues and corneal endothelial cells and which is required to sustain those functions. Further, a pH of 7.2 to 8.0 is more preferable, and the range has been found to be permissible as an irrigating solution for ocular surgery as the result of the efficacy and safety tests using the eyes of rabbits. To have the above range of pH, the above buffer comprising bicarbonates and citrate ions is preferable. In addition, an acetic acid-based buffer, a boric acid-based buffer, and a tris(hydroxy)aminomethane-hydrochloric acid-based buffer can also be used. The present invention will be described in detail with reference to Examples hereinafter.

### Examples

### Example 1

Irrigating solutions Nos. 1 to 7 for testing were prepared by dissolving the predetermined amounts of the components listed in Table 1 and, finally, sodium D-3-hydroxybutyric acid (to be referred to as "D-3-HBA·Na" hereinafter) in distilled water so as to adjust the total amounts of the solutions to be 1 liter each; adjusting the pHs of the solutions by diluted hydrochloric acid as required, and subjecting the solutions to aseptic filtration. Further, Comparative Solutions 2 to 5 were prepared in the same manner as described above, and the components of Commercial Product B (Comparative Solution 1) used as Comparative Example and the concentrations of the components are also shown in Table 1.

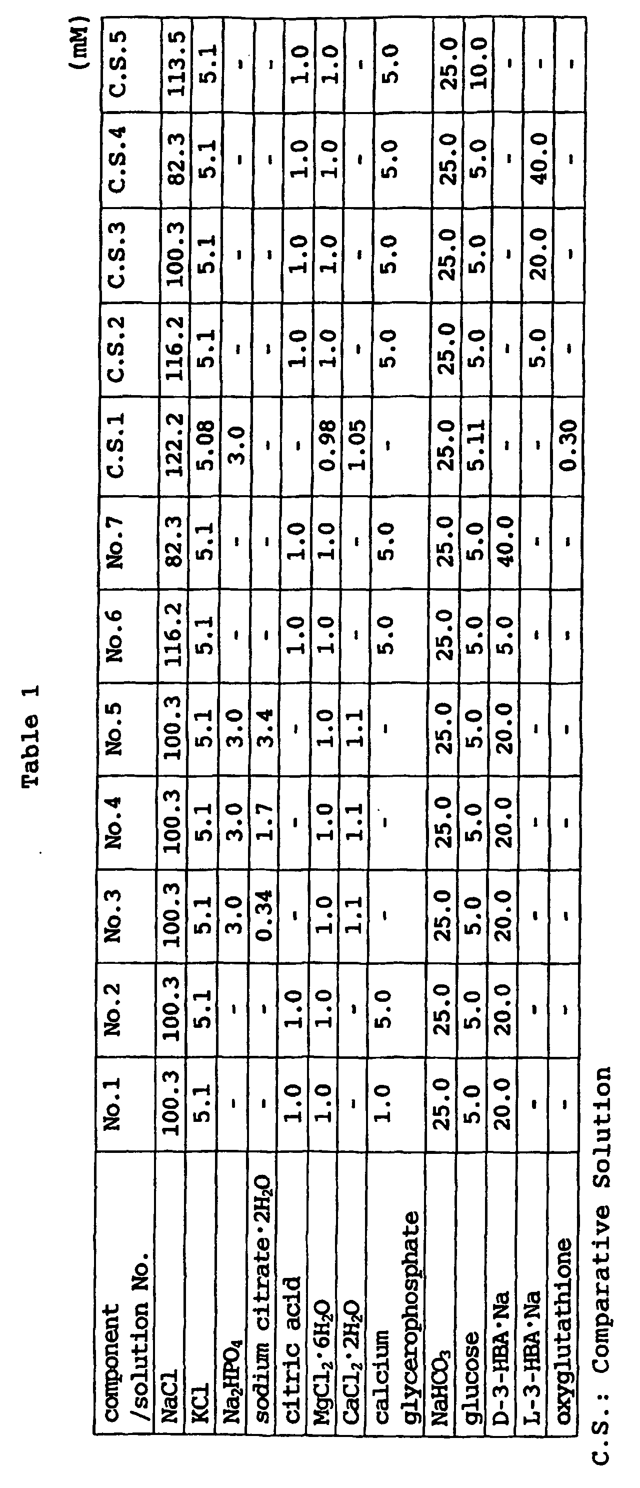

### Example 2

A preparation stability test was performed by using the Solutions Nos. 1 to 5 of Example 1. Five bottles of the solution to be tested were prepared by charging 500 mL of the preparation into 600mL transparent glass containers and capping the bottles. Those were preserved for 6 months in a constant-temperature and constant-humidity instrument maintained at 40°C±0.5°C and a relative humidity of 75%± 5%. The appearances, insoluble matter tests, osmotic pressures and pHs of the solutions were evaluated in accordance with the Japanese pharmacopeia, thirteenth Edition. As a result, no changes occurred in the appearances of the solutions the insoluble matter tests and the osmotic pressures. As for the pHs of the solutions, while they were 7.43±0.02 at the start of the preservation, they became 7.52±0.02 after the 6-month preservation, showing relatively small changes. Accordingly, it was found that the preparations could be preserved stably at room temperature for the long term.

### Example 3

To evaluate the preparation stability under severe temperature conditions, the appearances of the testing solutions prepared in the same manner as in Example 2 were evaluated. These were preserved in a constant-temperature instrument kept at 60°C for one month and evaluated after one-week preservation and after one-month preservation. As a result, no precipitation was observed in the solutions No. 1 and No. 2 after the one-month preservation, while precipitation was observed in the solutions Nos. 3 to 5 after the one-week preservation, and the amount of the precipitation was increased after the one-month preservation. Therefore, it was found that the solutions No. 1 and No. 2 of the present invention were excellent in stability under severe temperature conditions.

### Example 4

Using the Solutions No. 2, No. 6 and No. 7 and Comparative Solutions 2 to 4 as described in Example 1, their cornea-protecting effects were compared with one another by using the corneal thickness-adjusting function of a corneal endothelial cell (in vitro experiment).

Corneoscleral pieces having about 5mm-wide sclerae around the corneas were extracted from mature rabbits and incubated in the testing irrigating solutions at 36° C for 6 hours. The corneal thicknesses after the 6-hour incubation were measured by an ultrasonic pachymeter (DGH-500 PACHETTE, a product of DGH TECHNOLOGY).

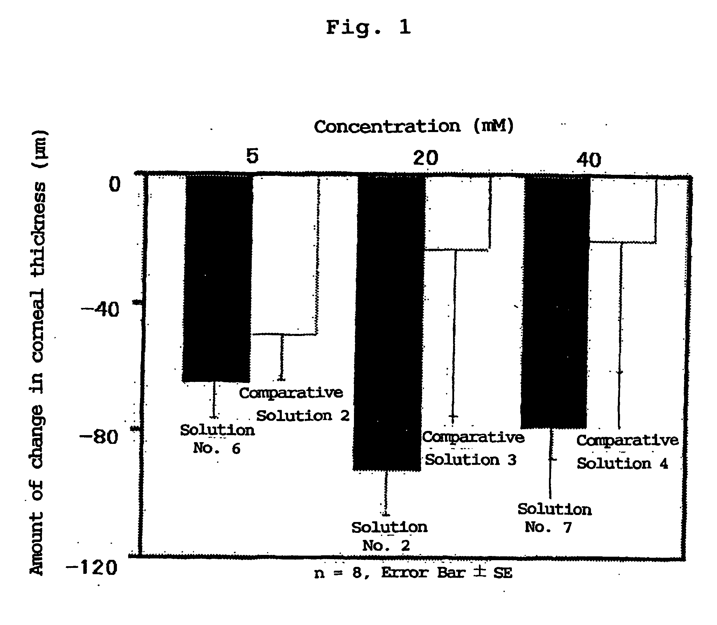

The amount of change in the corneal thickness after the 6-hour incubation by each testing solution are shown in Fig. 1. The amount of change in the corneal thickness was indicated by a negative value when the corneal thickness after the incubation was decreased to smaller than that before the incubation and by a positive value when the corneal thickness after the incubation was increased to larger than that before the incubation.

As for the amount of change in the corneal thickness by each of the testing solutions containing the D-form or the L-form, the cells incubated by the all of testing solutions containing D-3-HBA·Na were kept with the corneal thickness further reduced. Of the testing solutions, the Solution No. 2 containing 20 mM of D-3-HBA·Na exhibited the most prominent cornea-protecting effect.

### Example 5

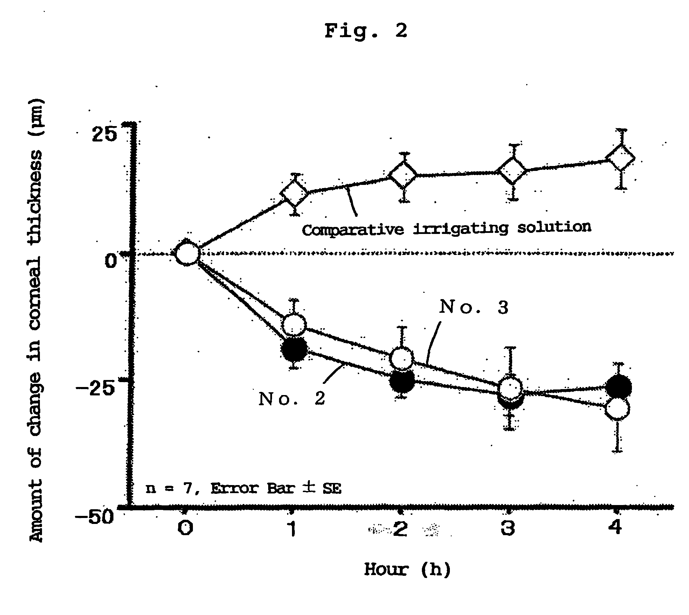

Using three types of testing irrigating solutions, i.e., the Solutions No. 2 and No. 3 described in Example 1 and a comparative irrigating solution having the same composition as that of the Solution No. 2 except for the absence of phosphate ions and glycerophosphate ions and having its osmotic pressure adjusted to the same level as those of the Solutions No. 2 and No. 3, their cornea-protecting effects were compared with one another by using the corneal thickness-adjusting function of a corneal endothelial cell (in vitro experiment) in the same manner as in Example 4 (except that only the incubation time was changed). The results are shown in Fig. 2.

As a result, it was found that the glycerophosphate ions served the same function as that of the phosphate ions which are the essential components of aqueous humor.

### Example 6

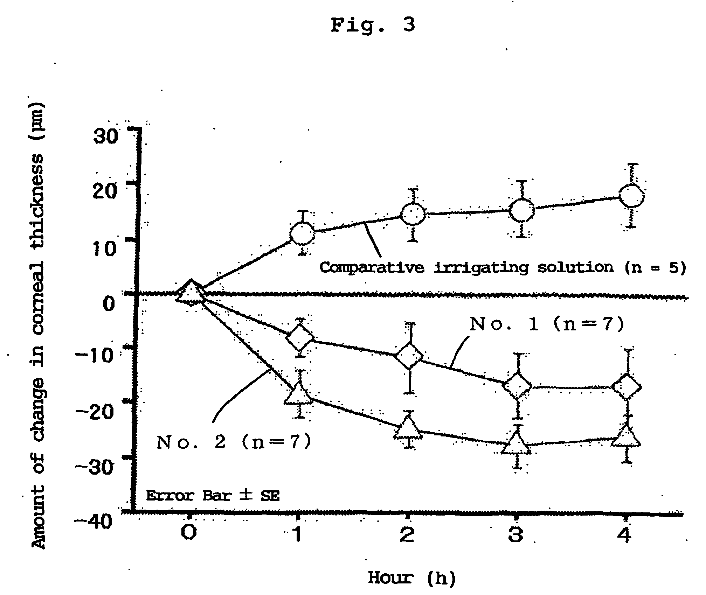

Using three types of testing irrigating solutions, i.e., the Solutions No. 1 and No. 2 described in Example 1 and a comparative irrigating solution having the same composition as that of the Solution No. 2 except for the absence of glycerophosphate ions and having its osmotic pressure adjusted to the same level as those of the Solutions No. 1 and No. 2, their cornea-protecting effects were compared with one another by using the corneal thickness-adjusting function of a corneal endothelial cell (in vitro experiment) in the same manner as in Example 5. The results are shown in Fig. 3.

As a result, it was found that the corneal thickness-adjusting function did not function in the absence of calcium glycerophosphate and that as the content of calcium glycerophosphate increased, that is, with the Solution No. 2 rather than the Solution No. 1, the corneal thickness-adjusting function functioned more effectively.

### Example 7

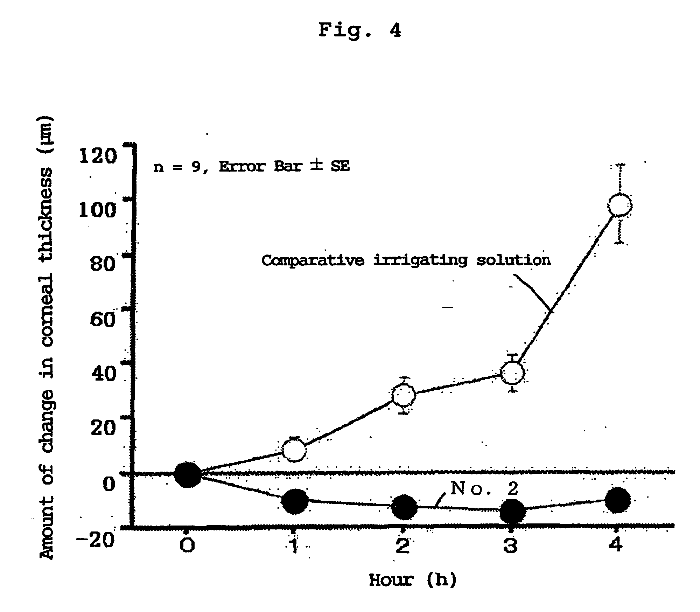

Using two types of testing irrigating solutions, i.e., the Solution No. 2 described in Example 1 and a comparative irrigating solution having the same composition as that of the Solution No. 2 except for the absence of bicarbonate ions and having its osmotic pressure adjusted to the same level as that of the Solution No. 2, their cornea-protecting effects were compared with each other by using the corneal thickness-adjusting function of a corneal endothelial cell (in vitro experiment) in the same manner as in Example 5. The results are shown in Fig. 4.

As a result, it was found that the corneal thickness-adjusting function did not function normally in the absence of bicarbonate ions and that the bicarbonate ions must therefore be contained.

### Example 8

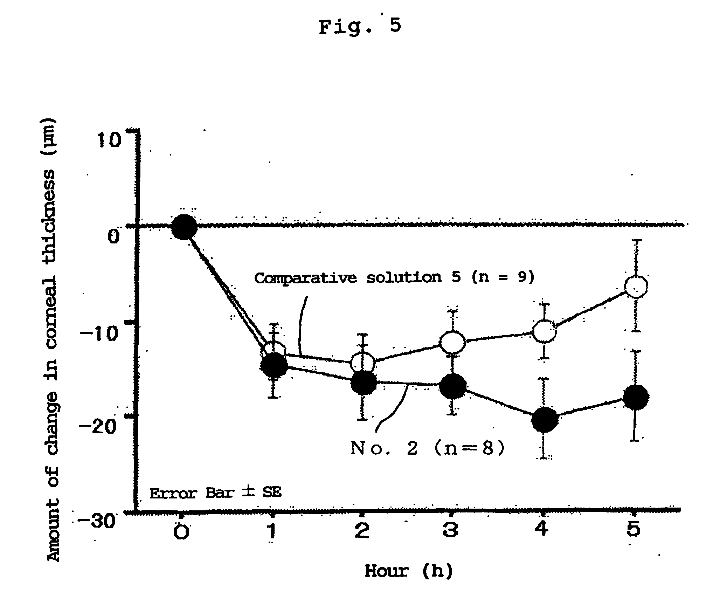

Using two types of testing irrigating solutions, i.e., the Solution No. 2 and the Comparative Solution 5 described in Example 1, their cornea-protecting effects were compared with each other by using the corneal thickness-adjusting function of a corneal endothelial cell (in vitro experiment) in the same manner as in Example 5. The results are shown in Fig. 5.

As a result, it was found that the corneal thickness-adjusting function functioned more effectively with the solution (No. 2) containing a combination of D-3-HBA·Na and glucose as an energy source than with the solution (Comparative Solution 5) containing only glucose as an energy source.

### Example 9

Two types of the testing irrigating solutions described in Example 1, i.e., the testing irrigating solution No. 2 and Comparative Solution 1, were examined for their effects to the corneal thickness by an in vivo experiment in which the solutions were irrigated in the anterior chambers of Dutch rabbits (male and female weighing 1.9 to 2.9 kg).

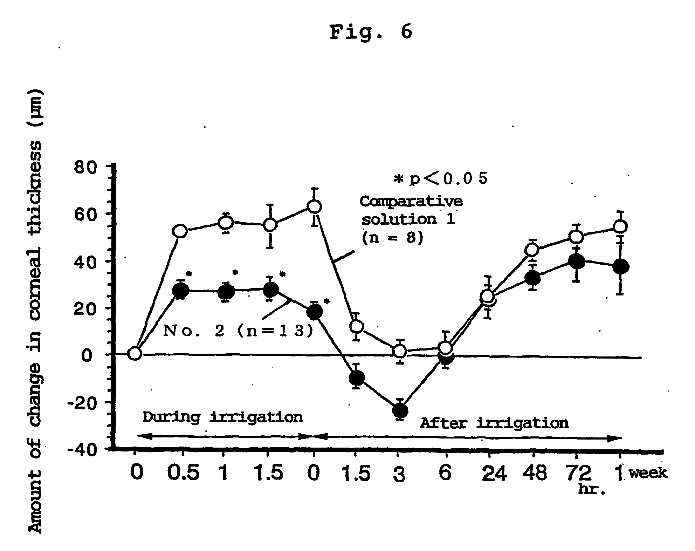

After putting the rabbits under general anesthesia by injecting xylazine hydrochloride and ketamine hydrochloride through intramuscular injection, a 3.2-mm-long incision was made around the corneas of the left and right eyes of each rabbit using a scalpel for ocular surgery. Subsequently, 18-gauge injection needles having a rounded tip were inserted in the anterior chambers through the incisions, and the two types of testing irrigating solutions were allowed to irrigate at a flow rate of 10 ml/min for 120 minutes. The amount of change in the corneal thickness by each solution was measured by the above ultrasonic pachymeter before the start of the irrigation and every 30 minutes until 120 minutes after the start of the irrigation under local anesthesia maintained by giving eye drops of a 0.4% oxybuprocaine hydrochloride solution. The amounts of changes in the corneal thicknesses with time are shown in Fig. 6.

As a result, it was found that the Solution No. 2, which was the present invention, caused a significantly small change in the corneal thickness especially during irrigation and had an excellent cornea-protecting effect, as compared with the Comparative Solution No. 1, which was a commercially available irrigating solution preparation.

### Example 10

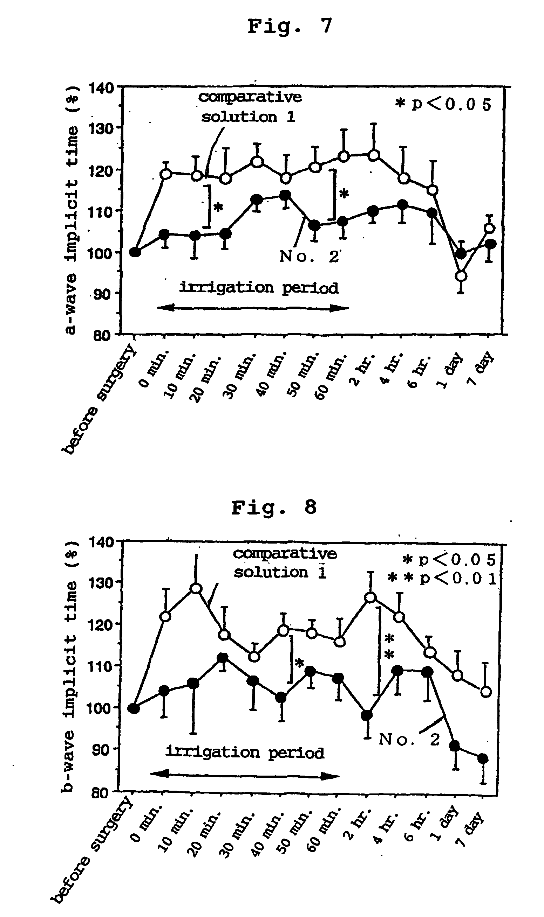

Using two types of testing irrigating solutions, i.e., the Solution No. 2 and Comparative Solution No. 1 described in Example 1, an electrophysiologic examination by ERG was conducted in the following experiment procedure in order to study the influences of the irrigating solutions on the retina extracted from a rabbit. The retina extracted from the rear portion of the eye ball of a rabbit was set in a chamber in which the retina was to be reacted with the testing solutions. By allowing a gas comprising 95% of O₂ and 5% of CO₂ to pass through the solutions, they were substituted by standard irrigating solutions at 35° C. After the retina was incubated for 60 minutes, the irrigating solutions were again substituted by standard irrigating solutions and the retina was incubated for another 60 minutes. The a-wave and b-wave implicit time (time until the reaction occurs) of ERG were then observed at intervals of 10 minutes (reference time: 0 minute). The rate of change in each implicit time of ERG was based on the implicit time immediately before the substitution of the testing solutions. The change of amplitude in each measuring time was converted into a percentage. The change with time of the a wave is shown in Fig. 7, and the change with time of the b wave is shown in Fig. 8.

As a result, it was found that the Solution No. 2, which was the solution of the present invention exerted a smaller influence on the retina than the Comparative Solution 1, which was a commercially available irrigating solution preparation.

ERG is one of the methods which are effective in acquiring general information about the retina, including a change in the retinal function, that is, the degree of reduction in the number of retinal cells, in other words, the range of seriousness of its symptom, the degree of qualitative and quantitative abnormalities of a visual substance and a neurotransmitter, the abnormalities of retinal cells and a cell membrane potential, and the degree of degeneration of a cell membrane (Ophthalmology Coherent Structure, Vol. 1, pp. 69-70, published by NAKAYAMA SYOTEN, 1993).

As described above, the present invention provides a one solution-pack irrigating solution for ocular surgery, which has a high living body safety and is stable as a preparation under severe temperature conditions, by using a combination of at least one compound selected from the group consisting of D-3-hydroxybutyric acid and its salts as an energy source, bicarbonates and calcium glycerophosphate. Further, the preparation of irrigating solution for ocular surgery has an excellent effect of protecting ocular tissues including corneal endothelial cells and an excellent effect of recovering physically damaged ocular tissues.

## Claims

1. A one solution-pack irrigating solution preparation for ocular surgery, which comprises
(i) at least one compound selected from the group consisting of D-3-hydroxybutyric acid and its salts,
(ii) bicarbonates, and
(iii) calcium glycerophosphate.

2. The preparation of claim 1, which further comprises glucose.

3. The preparation of claim 1, which further comprises at least one compound selected from the group consisting of citric acid and its salts.

4. The preparation of claim 1, wherein D-3-hydroxybutyric acid and its salts are selected from the group consisting of sodium salts, potassium salts, barium salts, magnesium salts, lithium salts, L-lysine salts, L-histidine salts and L-arginine salts.

5. The preparation of claim 1, wherein the bicarbonates are sodium salts and potassium salts.

6. The preparation of claim 1, wherein the concentration of at least one compound selected from the group consisting of D-3-hydroxybutyric acid and its salts is at least 0.01 mM but less than 200 mM.

7. The preparation of claim 1, wherein the concentration of the bicarbonates is at least 0.1 mM but less than 100 mM.

8. The preparation of claim 1, wherein the concentration of calcium glycerophosphate is at least 0.1 mM but less than 50 mM.

9. The preparation of claim 2, wherein the concentration of glucose is at least 0.01 mM but less than 50 mM.

10. The preparation of claim 3, wherein the concentration of at least one compound selected from the group consisting of citric acid and its salts is at least 0.01 mM but less than 50 mM.

11. The preparation of claim 1, which further comprises at least one compound selected from the group consisting of inorganic salts as biological components and isotonic agents.

12. The one solution-pack irrigating solution preparation of claims 1, 2, 3 and 11 for use in the surgery of cataract, intraocular lens implantation, glaucoma, vitrectomy body or penetrating keratoplasty.

## Patentansprüche

1. Irrigationslösungszubereitung mit Einzellösungspackung zur Augenoperation, die umfasst
(i) mindestens eine Verbindung, ausgewählt aus der Gruppe, bestehend aus D-3-Hydroxybuttersäure und ihren Salzen,
(ii) Bicarbonate und
(iii) Calciumglycerophosphat.

2. Zubereitung nach Anspruch 1, die außerdem Glucose umfasst.

3. Zubereitung nach Anspruch 1, die außerdem mindestens eine Verbindung, ausgewählt aus der Gruppe, bestehend aus Citronensäure und ihren Salzen, umfasst.

4. Zubereitung nach Anspruch 1, wobei D-3-Hydroxybuttersäure und ihre Salze aus der Gruppe ausgewählt sind, bestehend aus Natriumsalzen, Kaliumsalzen, Bariumsalzen, Magnesiumsalzen, Lithiumsalzen, L-Lysinsalzen, L-Histidinsalzen und L-Argininsalzen.

5. Zubereitung nach Anspruch 1, wobei die Bicarbonate Natriumsalze und Kaliumsalze sind.

6. Zubereitung nach Anspruch 1, wobei die Konzentration von mindestens einer Verbindung, ausgewählt aus der Gruppe, bestehend aus D-3-Hydroxybuttersäure und ihren Salzen, mindestens 0,01 mM, allerdings weniger als 200 mM beträgt.

7. Zubereitung nach Anspruch 1, wobei die Konzentration der Bicarbonate mindestens 0,1 mM, allerdings weniger als 100 mM beträgt.

8. Zubereitung nach Anspruch 1, wobei die Konzentration an Calciumglycerophosphat mindestens 0,1 mM, allerdings weniger als 50 mM beträgt.

9. Zubereitung nach Anspruch 2, wobei die Konzentration an Glucose mindestens 0,01 mM, allerdings weniger als 50 mM beträgt.

10. Zubereitung nach Anspruch 3, wobei die Konzentration von mindestens einer Verbindung, ausgewählt aus der Gruppe, bestehend aus Citronensäure und ihren Salzen, mindestens 0,01 mM, allerdings weniger als 50 mM beträgt.

11. Zubereitung nach Anspruch 1, die außerdem mindestens eine Verbindung aufweist, ausgewählt aus der Gruppe, bestehend aus anorganischen Salzen als biologische Komponenten und isotonischen Mitteln.

12. Irrigationslösungszubereitung mit Einzellösungspackung nach den Ansprüchen 1, 2, 3 und 11 zur Verwendung bei der Kataraktoperation, intraokularen Linsenimplantation, Glaukom, Glaskörperentfernung oder penetrierender Keratoplastik.

## Revendications

1. Préparation de solution d'irrigation à un composant en solution pour la chirurgie oculaire, qui comprend
(i) au moins un composé choisi dans le groupe consistant en l'acide D-3-hydroxybutyrique et ses sels,
(ii) des bicarbonates et
(ii) du glycérophosphate de calcium.

2. Préparation suivant la revendication 1, qui comprend, en outre, du glucose.

3. Préparation suivant la revendication 1, qui comprend, en outre, au moins un composé choisi dans le groupe consistant en l'acide citrique et ses sels.

4. Préparation suivant la revendication 1, dans laquelle l'acide D-3-hydroxybutyrique et ses sels sont choisis dans le groupe consistant en des sels de sodium, des sels de potassium, des sels de baryum, des sels de magnésium, des sels de lithium, des sels de L-lysine, des sels de L-histidine et des sels de L-arginine.

5. Préparation suivant la revendication 1, dans laquelle les bicarbonates sont des sels de sodium et des sels de potassium.

6. Préparation suivant la revendication 1, dans laquelle la concentration d'au moins un composé choisi dans le groupe consistant en l'acide D-3-hydroxybutyrique et ses sels est d'au moins 0,01 mM, mais est inférieure à 200 mM.

7. Préparation suivant la revendication 1, dans laquelle la concentration des bicarbonates est d'au moins 0,1 mM, mais est inférieure à 100 mM.

8. Préparation suivant la revendication 1, dans laquelle la concentration de glycérophosphate de calcium est d'au moins de 0,1 mM, mais est inférieure à 50 mM.

9. Préparation suivant la revendication 2, dans laquelle la concentration de glucose est d'au moins 0,01 mM, mais est inférieure à 50 mM.

10. Préparation suivant la revendication 3, dans laquelle la concentration d'au moins un composé choisi dans le groupe consistant en l'acide citrique et ses sels est d'au moins 0,01 mM, mais est inférieure à 50 mM.

11. Préparation suivant la revendication 1, qui comprend, en outre, au moins un composé choisi dans le groupe consistant en des sels minéraux en tant que constituants biologiques et agents isotoniques.

12. Préparation d'une solution d'irrigation en un composant en solution suivant les revendications 1, 2, 3 et 11 à utiliser dans la chirurgie de la cataracte, dans l'implantation de lentilles intraoculaires, dans le glaucome, dans la vitrectomie ou dans la kératoplastie pénétrante.
